# EUROPEAN PATENT APPLICATION

(11) **EP 1 914 217 A1**
(43) Date of publication of application: **23.04.2008**
(21) Application number: 06782227.0
(22) Date of filing: 03.08.2006
(51) Int. Cl.: C07C 17/08, C07C 22/04

(54) **PROCESS FOR PRODUCTION OF CHLORINATED AROMATIC COMPOUND**

(30) Priority: 08.08.2005 JP 2005229403; 17.11.2005 JP 2005332518
(71) Applicant: Kaneka Corporation, Kita-ku Osaka-shi, Osaka 530-8288 (JP)
(72) Inventor: KYOTANI, Susumu, Takasago-shi, Hyogo; 6768688 (JP); FURUKAWA, Naoki, Takasago-shi, Hyogo; 6768688 (JP)
(74) Representative: Vossius & Partner
(86) International application number: PCT/JP2006/315367
(87) International publication number: WO 2007/018109

(57) **Abstract**

[PROBLEMS] To provide a simpler method for producing a high-quality chlorinated aromatic hydrocarbon (e.g., cumylchloride), which can be used as a cationic polymerization initiator.

[MEANS FOR SOLVING PROBLEMS] A chlorinated aromatic hydrocarbon can be produced by repeating both (a) the step of performing chlorination reaction by stirring an organic solution containing a compound represented by Ar(R¹C=CH₂)ₙ with an aqueous hydrochloric acid solution having a hydrochloric acid concentration of 30% by weight or higher and (b) the step of removing the water phase in part or whole from the reaction solution produced in the step (a), and then adding an aqueous hydrochloric acid solution having a hydrochloric acid concentration of 30% by weight or higher to the reaction solution, until the chlorination reaction rate of the compound reaches a predetermined level.

## Description

### TECHNICAL FIELD

The present invention is a novel method for easily and efficiently producing a chlorinated aromatic hydrocarbon.

### BACKGROUND ART

There has been known that chlorinated aromatic compounds such as cumylchloride (C₆H₅C(CH₃)₂Cl) and dicumylchloride (1,4-Cl(CH₃)₂CC₆H₄C(CH₃)₂Cl) are used as an initiator for producing a block copolymer, such as isobutylene styrene copolymer, having terminal functional polyisobutylene or polyisobutylene as a block component through a cationic polymerization (Patent Documents 1 and 2).

There has been known that such an initiator is synthesized by performing reaction in which hydrogen chloride is added to α-methylstyrene or 1,4-diisopropenylbenzene cooled with ice (Patent Documents 1 and 2).

However, in the above method, gas such as hydrogen chloride or chlorine is used as a reagent for chlorination. Therefore, this production involves a gas-liquid reaction. Therefore, a reaction condition such as stirring efficiency has a considerable influence on a yield. In addition, the above method has the problem that it needs a largely excess chlorinating reagent stoichiometrically. Furthermore, ice cooling is needed in the reaction. Therefore, the above method is not industrially advantageous. As a result, a highly efficient and simple production method has been required.
(Patent Document 1) U.S. Pat. No. 4,946,899 Specification
(Patent Document 2) U.S. Pat. No. 4,276,394 Specification
(Non-Patent Document 1) Journal of American Chemical Society by H.C. Brown and Min-Hon Rei, Vol. 31, P.1090-1093 (1966).
(Non-Patent Document 2) Makromol. Chem. by O. Nuyken, S.D. Pask, A. Vischer and M. Walter, Vol. 186, P. 173-190 (1985).

### DISCLOSURE OF INVENTION

### [Problems to be solved by the Present Invention]

In view of the current situation, an object of the present invention is to provide a simple and efficient method for producing a high-quality chlorinated aromatic compound, such as cumylchloride and dicumylchloride, which can be used as a cationic polymerization initiator.

### [Means for solving the Problem]

The invention achieved to solve the above problem relates to a method for producing a chlorinated aromatic compound represented by general formula (2):

Ar(R¹CCH₃Cl)ₙ (2)

where Ar is an n-valent aromatic ring group, R¹ is a substituted or unsubstituted univalent aliphatic hydrocarbon group, and n is an integer from 1 to 5, including the steps of: (a) performing chlorination reaction by stirring, (i) an organic solution containing a compound represented by general formula (1):

Ar(R¹C=CH₂)ₙ (1)

where Ar, R¹, and n are the same as those in general formula (2) and (ii) an aqueous hydrochloric acid solution having a hydrochloric acid concentration of 30% by weight or higher; (b) removing a water phase in part or whole from a reaction solution produced in the step (a) and then adding another aqueous hydrochloric acid solution having a hydrochloric acid concentration of 30% by weight or higher to the reaction solution, the steps (a) and (b) being repeated until a chlorination reaction rate of the compound represented by the general formula (1) reaches a predetermined level.

A preferable embodiment is a method for producing a chlorinated aromatic compound represented by general formula (2):

Ar(R¹CCH₃Cl)ₙ (2)

where Ar is an n-valent aromatic ring group, R¹ is a substituted or unsubstituted univalent aliphatic hydrocarbon group, and n is an integer from 1 to 5, wherein when a hydrochloric acid concentration of a water phase of the reaction solution becomes less than 30% by weight after the step (a), the water phase is removed in part or whole, and then another aqueous hydrochloric acid solution is added to the reaction solution so that a hydrochloric acid concentration of the water phase in the reaction solution becomes 30% by weight or higher. In addition, it is preferable that the addition of the aqueous hydrochloric acid solution is repeated until the chlorination reaction rate reaches 95mol% or more. Furthermore, a hydrogen chloride gas may be used additionally for chlorination.

In addition, another preferable embodiment is a method for producing the chlorinated aromatic compound represented by general formula (2):

Ar(R¹CCH₃Cl)ₙ (2)

where Ar is an n-valent aromatic ring group, R¹ is a substituted or unsubstituted monovalent aliphatic hydrocarbon group, and n is an integer from 1 to 5, including the successive steps of: (a) performing chlorination reaction by mixing (i) an organic solution containing a compound represented by general formula (1):

Ar(R¹C=CH₂)ₙ (1)

where Ar, R¹, and n are the same as those in general formula (2) and (ii) an aqueous hydrochloric acid solution having a hydrochloric acid concentration of 30% by weight or higher; (b) removing a water phase of the thus obtained reaction solution; and (c) bringing an oil phase of the reaction solution into contact with hydrogen chloride gas.

Another preferable embodiment is a method for producing the chlorinated aromatic compound, wherein: the compound represented by the general formula (1) is α-methylstyrene, 1,4-diisopropenylbenzene, 1,3-diisopropenylbenzene, 1,2-diisopropenylbenzene, 1,3,5-triisopropenylbenzene, or (1,3-diisopropenyl-5-tert-butyl) benzene.

### [Advantageous Effect of the Present Invention]

According to the method of the present invention, it is possible to more easily and efficiently obtain a high-quality chlorinated aromatic compound, such as cumylchloride and dicumylchloride, which can be used as a cationic polymerization initiator, in comparison with the conventional method using hydrogen chloride gas or chlorine gas.

### BEST MODE FOR CARRYING OUT THE INVENTION

In the present invention, an organic solution containing a compound represented by general formula (1):

Ar(R¹C=CH₂)ₙ (1)

where Ar represents an n-valent aromatic ring group, R¹ represents a substituted or unsubstituted univalent aliphatic hydrocarbon group, n is an integer from 1 to 5 is stirred with an aqueous hydrochloric acid solution having a hydrochloric acid concentration of 30% by weight or higher, so that the compound represented by general formula (1) is chlorinated (chlorination reaction step).

The chlorination reaction occurs as follows: That is, an organic solution containing a compound represented by the general formula (1) is stirred with an aqueous hydrochloric acid solution having the hydrochloric acid concentration of 30% by weight or higher, thereby to obtain a reaction solution. Next, a water phase is removed in part or whole from the reaction solution. Thereafter, another aqueous hydrochloric acid solution having a hydrochloric acid concentration of 30% by weight or higher is added to the reaction solution having a compound represented by the general formula (1). Then, the resulting reaction solution is further stirred. The chlorination reaction step and the step of removing the water phase from the reaction solution and then adding the aqueous hydrochloric acid solution to the reaction solution, are repeatedly carried out until the chlorination reaction rate of the compound represented by the general formula (1) reaches an intended level. In view of reaction efficiency, the addition of the aqueous hydrochloric acid solution is preferably repeatedly carried out until the chlorination reaction rate reaches 95mol% or higher. In addition, in the case where the hydrochloric acid concentration of the solution under the chlorination reaction decreases to less than 30% by weight, it is preferable to remove a water phase in part or whole from the reaction solution and then add another aqueous hydrochloric acid solution to the solution until a hydrochloric acid concentration of the water phase reaches 30% by weight or higher. It is preferable to carry out this operation repeatedly until the chlorination reaction rate reaches a predetermined level.

As a result, it is possible to efficiently produce a chlorinated aromatic compound represented by general formula (2):

Ar(R¹CCH₃Cl)ₙ (2)

where Ar, R¹, and n are the same as those in the general formula (1). Meanwhile, in view of production efficiency, it is preferable that the final chlorination reaction rate is 95mol% or higher.

The aromatic ring group represented by Ar in the compounds represented by the general formula (1) and the general formula (2) is, for example, C₆H₅-, p-C₆H₄-, m-C₆H₄-, o-C₆H₄-, and a 1,3,5-C₆H₃-group. R¹ and R² are hydrocarbon groups such as a methyl group and an ethyl group. R¹ and R² may have a substituent such as a chlorine atom. Examples of such compounds are α-methylstyrene, 1,4-diisopropenylbenzene, 1,3-diisopropenylbenzene, 1,2-diisopropenylbenzene, 1,3,5-triisopropenylbenzene, and (1,3-diisopropenyl-5-tert-butyl) benzene.

In the compounds represented by the general formula (1) and the general formula (2), n is an integer from 1 to 5 and chosen appropriately according to a required polymer structure and properties.

The concrete examples of the chlorinated aromatic compounds produced by using the method of the present invention are
(1-chlor-1-methylethyl)benzene[C₆H₅C(CH₃)₂Cl],
1,4-bis(1-chlor-1-methylethyl)benzene[1,4-Cl(CH₃)₂CC₆H₄C (CH₃)₂Cl],
1,3-bis(1-chlor-1-methylethyl)benzene[1,3-Cl(CH₃)₂CC₆H₄C (CH₃)₂Cl],
1,2-bis(1-chlor-1-methylethyl)benzene(1,2-Cl(CH₃)₂CC₆H₄C (CH₃)₂Cl),
1,3,5-tris(1-chlor-1-methylethyl)benzene[1,3,5-(ClC(CH₃)₂) ₃C₆H₃], and 1,3-bis(1-chlor-1-methylethyl)-5-(tert-butyl) benzene[1,3-(C(CH₃)₂Cl)₂-5-(C(CH₃)₃)C₆H₃].

In the present invention, a mixture of a vinyl-group-containing aromatic compound represented by the above general formula (1) and an organic solvent may be stirred with an aqueous hydrochloric acid solution. Alternatively, an aqueous hydrochloric acid solution may be stirred directly with a vinyl-group-containing aromatic compound represented by the above general formula (1) in a case where the vinyl-group-containing aromatic compound is in the form of a liquid.

Any conventionally known organic solvent can be used for the present reaction. Examples of the organic solvent include: saturated hydrocarbons such as pentane, cyclopentane, neopentane, hexane, cyclohexane, heptane, methylcyclohexane, octane, norbornene, and ethyl cyclohexane; aromatic hydrocarbons such as benzene, toluene, xylene, and ethyl benzene; halogenated hydrocarbons such as carbon tetrachloride, chloroform, methylene chloride, chloroethane, dichloroethane, propyl chloride and butyl chloride; ketones such as acetone, methyl ethyl ketone, and diethyl ketone; ethers such as diethyl ether, diisopropyl ether, dibutyl ether, and dimethoxyethane; alcohols such as methanol, ethanol, isopropanol, and butanol; dimethylformamide; dimethyl sulfoxide; and HMPA.

Specifically, the organic solvent is preferably a saturated hydrocarbon or an aromatic hydrocarbon for the reason that hydrochloric acid is less soluble therein and oil-water separation of these solvents is easy. The organic solvent is more preferably pentane, cyclopentane, neopentane, hexane, cyclohexane, heptanes, methylcyclohexane, octane, norbornene, ethyl cyclohexane, benzene, toluene, xylene, and ethyl benzene due to its low mutual solubility in water. In addition, the halogenated hydrocarbons, which are industrially used as solvents for polymerization, can be used suitably for the reaction.

The quantity of the solvent for use in the reaction is not particularly limited. However, in view of handling after the reaction, the quantity of the solvent is preferably zero to ten times, more preferably zero to three times heavier than the raw material by weight.

An order of adding the aqueous hydrochloric acid solution can be changed if necessary due to manufacturing constraints and the like. Specifically, a compound represented by the general formula (1) may be added to the aqueous hydrochloric acid solution. Alternatively, an organic solution containing a compound represented by the above general formula (1) may be added to the aqueous hydrochloric acid solution.

In the present invention, an aqueous hydrochloric acid solution having a hydrochloric acid concentration of 30% by weight or higher is used, so that the hydrochloric acid concentration of a water phase in the reaction solution can be 30% by weight.

Furthermore, the aqueous hydrochloric acid solution in a required quantity is not added at one time, but added separately in several times, as described above. Specifically, the hydrochloric acid concentration of a water phase in the reaction solution decreases as the chlorination reaction proceeds. In this situation, the water phase is removed in part or whole from the reaction solution, and then another aqueous hydrochloric acid solution is added to the reaction solution. The aqueous hydrochloric acid solution can be added at a regular time interval, so that reaction efficiency improves. As a result, the chlorination can be carried out by a smaller amount of a hydrochloric acid than in the conventional method. The replacement frequency of the aqueous hydrochloric acid solution is not particularly limited. The aqueous hydrochloric acid solution is replaced repeatedly until the chlorination reaction rate reaches a predetermined level. In addition, in the case where a hydrochloric acid concentration of the water phase of the stirred reaction solution decreases to less than 30% by weight, it is preferable that another aqueous hydrochloric acid solution is added to the reaction solution after the water phase is removed in part or whole from the reaction solution.

A temperature at which the aqueous hydrochloric acid solution is reacted with the compound represented by the general formula (1) is preferably 0 to 50°C, more preferably 0 to 40°C in view of the rate of reaction and the stability of a target substance. If the temperature condition is below 0°C, the rate of the reaction slows down, and crystallization of the target product may occur. As a result, a reaction system becomes inhomogeneous. Furthermore, the reaction temperature above 50°C is unfavorable in view of corrosion of a material for an apparatus and the control of the hydrochloric acid concentration.

In the reaction, an aqueous hydrochloric acid solution and a solution containing the compound represented by the general formula (1) are stirred together. The stirring can be performed under a condition in which an oil phase and a water phase are separated. However, the stirring is preferably performed under a condition in which a droplet of the oil phase exists as a dispersal phase in the water phase. The hydrochloric acid concentration can be measured by taking a small quantity of the reaction solution during the progression of the reaction, and then performing neutralizing titration on the water phase of the reaction solution. In addition, a chlorination reaction rate can be obtained by ¹HNMR analysis. Specifically, in the case where α-methylstyrene is chlorinated, a chlorination reaction rate can be obtained by the ratio of an integration value of methylproton (6H) in cumylchloride, which is a target product, to an integration value of vinylproton (2H) in the α-methylstyrene, which is a raw material.

A stirrer blade type is not particularly limited. The stirrer blade may be a paddle blade such as a pitched paddle and an inclined paddle, and a turbine blade. A Max Blend blade, which is recommended by Sumitomo Heavy Industries, Ltd., or a Full Zone blade, which is recommended by Shinko Pantec CO., LTD., can be used as a large stirrer blade. In addition, a baffle, which provides a good mixing, can be provided in a stirring vessel.

In the present invention, in view of purity improvement, after the water phase is removed in part or whole, a hydrogen chloride gas may be supplied so that the chlorination reaction rate can be accelerated. As long as the reactor has a vapor phase, it is difficult that all the gas put into the reactor reacts. Therefore, the quantity of the hydrogen chloride gas used in the reaction is difficult to be defined. However, the hydrogen chloride gas is consumed by the reaction in the solution in which the hydrogen chloride gas is dissolved or dispersed. It is preferable that the quantity of the consumed hydrogen chloride gas out of the total quantity of the hydrogen chloride gas put into the reactor is an equivalent or more in relative to an impurity such as an isopropenyl group. It is more preferable that the quantity of the consumed hydrogen chloride gas is two equivalents or more in relative to the impurity, so that the degree of purity is increased efficiently. In order to reduce the quantity of the impurity remaining, it is effective to accelerate the rate of the reaction by increasing the quantity of hydrogen chloride gas dissolved in the liquid, or to increase the partial pressure of the hydrogen chloride gas in the vapor phase.

In addition, the temperature at which the reaction is carried out is preferably in the range from 0 to 50°C for the same reason as in the reaction with the aqueous hydrochloric acid solution, and more preferably 0 to 40°C. In the above-described method in which after the reaction is performed by mixing the aqueous hydrochloric acid solution, hydrogen chloride gas is provided to cause further reaction, the replacement of the aqueous hydrochloric acid solution can be stopped before the chlorination reaction rate of the compound represented by the general formula (1) reaches a predetermined level, and then the hydrogen chloride gas is provided. In this case, an aqueous hydrochloric acid solution having the hydrochloric acid concentration of 30% by weight or lower can be used for the reaction.

A method for bringing an organic phase into contact with the hydrogen chloride gas is not particularly limited. The method can be a general operating method for a gas-liquid reaction, such as bubbling the hydrogen chloride gas or mixing the organic phase with a pressured hydrogen chloride gas in a stirring vessel.

In addition, in a case where hydrogen chloride gas is used for the reaction, it is preferable to perform a generally known degassing method, such as bubbling of inactive gas or decompression operation for the following reason. That is, when a chlorinated aromatic compound in the reaction solution that contains a target chlorinated aromatic is finally used as a polymerization initiator in the presence of the hydrogen chloride gas in the reaction solution, the quality of the resulting polymer may be deteriorated.

According to the method of the present invention, the reaction temperature can be set to be higher than that in the method described in Non-Patent Document 1 in which only a hydrogen chloride gas is used. A reaction needs to be performed at around 0°C in a reaction system in which a hydrogen chloride is used. However, according to the method of the present invention, a side reaction is preventable even at around room temperature. As a result, a target compound can be obtained with high yields. That is, according to the method of the present invention, it is possible to perform the reaction at 0°C or higher temperature. Furthermore, it is possible to increase the rate of the reaction when the reaction temperature is at 0 to 40°C. The increase of the reaction temperature eliminates for the need of cooling. This makes it possible to simplify the manufacturing facilities, thus lowering the cost of manufacturing.

### [Examples]

The examples of the present invention are described below. However, the present invention is not limited to the

### examples.

### (Example 1)

First, 50g of α-methylstyrene and 22g of a concentrated hydrochloric acid at a concentration of 36% by weight were placed in a stirrer-equipped flask so that the concentrated hydrochloric acid is an equivalent in relative to α-methylstyrene. Next, the mixture of α-methylstyrene and the concentrated hydrochloric acid was stirred under a temperature condition of 30°C for two hours. Thereafter, the stirring was stopped, and then an oil phase and a water phase were separated in a stationary state. Then, all of the water phase was removed by a pipette. Next, a half equivalent of another concentrated hydrochloric acid (11g of the concentrated hydrochloric acid at the concentration of 36% by weight) was added so that the reaction was carried on. Thereafter, a half equivalent of the concentrated hydrochloric acid in the reaction solution was repeatedly replaced with another half equivalent of the concentrated hydrochloric acid every hour. The reaction continued for seven hours in total. As a result, the hydrochloric acid concentration in the water phase was set to be 30% by weight or higher in the reaction. After the seven-hour reaction, the water phase was removed, and the hydrochloric acid was removed by nitrogen substitution. After that, a cumylchloride was obtained. The chlorination reaction rates measured per reaction time period are shown in Table 1.

### (Example 2)

A mixture solution containing the same substance contents as in Example 1 was stirred by using the same reaction apparatus as in Example 1 under a temperature condition of 30°C for two hours. Thereafter, the stirring was stopped, and an oil phase and a water phase ware separated in a stationary state. Next, all of the water phase was removed by a pippet. Then, a half equivalent of another concentrated hydrochloric acid was added so that the reaction was carried on. Thereafter, a half equivalent of the concentrated hydrochloric acid in the reaction was repeatedly replaced with another half equivalent of the concentrated hydrochloric acid every hour. The reaction was continued for four hours in total. As a result, the hydrochloric acid concentration in the water phase was set to be 30% by weight or higher in the reaction. After the four-hour reaction, the water phase was removed. After that, the organic phase was aerated for two hours with a hydrogen chloride gas introduced at a rate of 100cc/min. After the hydrogen chloride gas aeration, nitrogen substitution was performed. As a result, a cumylchloride was obtained. The chlorination reaction rates measured per reaction time period are shown in Table 1.

### (Example 3)

In the present example, the reaction was carried out for seven hours in total as in Example 1, except that a solution containing α-methylstyrene and a concentrated hydrochloric acid at a concentration of 36% by weight were mixed for an hour, after the one-hour mixing, the aqueous hydrochloric acid solution of the mixture was replaced with another aqueous hydrochloric acid solution, and then the concentrated hydrochloric acid was replaced with 22g of another concentrated hydrochloric acid at a concentration of 36% by weight every two hours. As a result, the hydrochloric acid concentration in the water phase was set to be 30% by weight or higher in the reaction. The chlorination reaction rates measured per reaction time period are shown in Table 1.

### (Comparative Example 1)

First, 50g of α-methylstyrene was placed in a reaction apparatus that is the same as in Example 1. Next, 88g of a concentrated hydrochloric acid at a concentration of 36% by weight (the total quantity of the concentrated hydrochloric acid that was added separately in Example 3) was added so that the concentrated hydrochloric acid is 4 equivalents in relative to the α-methylstyrene. Next, the α-methylstyrene and the concentrated hydrochloric acid were mixed under a temperature condition of 30°C for seven hours. The result is shown in Table 1. When the concentrated hydrochloric acid of which total quantity is equal to the quantity of the separately added concentrated hydrochloric acid in Example 3 was added at one time, a high-purity chlorinated aromatic compound, which could be obtained by using the separately-added concentrated hydrochloric acid, could not be obtained.

### (Comparative Example 2)

In this example, the reaction was performed under the same conditions as in Example 3, except that a concentrated hydrochloric acid at a concentration of 29% by weight was used. During the reaction, the hydrochloric acid concentration in the water phase was constantly lower than 30% by weight. The result is shown in Table 1. As is clear from Table 1, a high-purity chlorinated aromatic compound cannot be obtained by using the hydrochloric acid concentration having a low concentration even when the aqueous hydrochloric acid solution is repeatedly replaced.

### (Method of Analysis)

The progress of the reaction was confirmed by taking out a reaction solution and then measuring the concentration of the hydrochloric acid through neutralization titration of the water phase (the aqueous hydrochloric acid solution) in the reaction solution. Specifically, 2g of aqueous hydrochloric acid solution which was accurately weighted was diluted with purified water. Next, a phenolphthalein solution was added to the diluent, and the resulting mixture was titrated with an aqueous solution of 1N-sodium hydroxide. In addition, the reaction rates were calculated by NMR analysis.

**Table 1**

| | Example 1 | Example 2 | Example 3 | Comparative Example 1 | Comparative Example 2 |
|---|---|---|---|---|---|
| Reaction Temperature (°C) | 30 | 30 | 30 | 30 | 30 |
| Reaction Period: Hr | Reaction Rate: % | Reaction Rate: % | Reaction Rate: % | Reaction Rate: % | Reaction Rate: % |
| 1 | - | 37.3 | - | - | 21.2 |
| 2 | - | 47.1 | - | - | - |
| 3 | - | 67.8 | - | - | - |
| 4 | 90.1 | 82.3 | 80.0 | - | 80 |
| 5 | - | - | - | - | - |
| 6 | - | - | - | - | - |
| 7 | 98.0 | - | 97.0 | 81.0 | 82.0 |
| Addition of Hydrogen Chloride Gas | - | 98.6 | 98.0 | - | - |
| After Degasification of Chlorine Gas | - | 96.0 | 96.5 | - | - |

| | | | | | |
|---|---|---|---|---|---|
| The reaction rates were calculated by NMR analysis. | | | | | |

As is clear from the above description, the method of the present invention makes it possible to obtain a high-purity chlorinated aromatic compound more easily and more efficiently than the conventional method.

The chlorinated aromatic compound was used for polymerization reaction as described in the below examples. A predetermined amount of the cumylchloride obtained in Example 3 was weighed out, and the predetermined amount of the cumylchloride was used as a polymerization initiator without undergoing any refining treatments. The polymerization experiment was performed as below.

### (Polymerization Example 1)

First, 595mL of n-butylchloride and 66.1mL of n-hexane were placed in a reaction container. Next, the reaction container was placed in a dry-ice-ethanol bath so that the temperature of the mixed solution was cooled down to -50°C. Thereafter, 276mL (2.92mol) of an isobutylene monomer was placed in the reaction container. Then, 0.882g (0.0057mol) of the cumylchloride obtained in Example 3 and 0.53g (0.0057mol) of picoline were placed in the reaction container. After the ingredients were placed, the temperature of the dry-ice-ethanol bath was set to -75°C while the ingredients in the reaction container was stirred. At the point in time when the temperature in the reaction container became -70°C, 3.57g (0.0188mol) of TiCl₄, which is a polymerization catalyst, is added in the reaction container so that the reaction was started. 105 minutes after the addition of the polymerization catalyst, the polymerization solution was added to a large amount of methanol so that the polymer was isolated. After that, the solvent was removed so that the polymer was taken out. After that, the polymer was subjected to reduced-pressure drying at the temperature of 60°C all day and night so that the polymer was obtained.

Molecular weight and its distribution of the polymer products were measured by GPC analysis. According to the result of the GPC analysis, the number average molecular weight (Mn) was 32100 and the molecular weight distribution (Mw/Mn) was 1.18 (Mw: weight average molecular weight). As is apparent from the result, an excellent polymer with a narrow molecular weight distribution was obtained.

### (Polymerization Example 2)

An isobutylene was polymerized by the same operation as in Polymerization Example 1. After that, 37mL (0.32mol) of a styrene was added to the polymer, and the polymerization reaction was continued for 120 minutes. After the reaction was finished, the reaction solution was stirred into a large amount of water so that the reaction solution was washed. Next, an organic phase and a water phase were separated so that the catalyst was removed. Then, a volatile component of the organic phase was removed by the same evaporation operation as in Example 1. As a result, a polymer product was obtained.

Molecular weight and its distribution of the polymer products were measured by GPC analysis. The result of the GPC analysis was as follows: the number average molecular weight (Mn) and molecular weight distribution (Mw/Mn) of the polymer products after the isobutylene polymerization were 32900, and 1.16, respectively. The number average molecular weight (Mn) and molecular weight distribution (Mw/Mn) of the polymer products after the styrene polymerization was 39300 and 1.18, respectively. As is apparent from the result, an excellent isobutylene-styrene block copolymer with a narrow molecular weight distribution was obtained.

As is clear from the above descriptions, by using a high-purity chlorinated aromatic compound obtained by the method of the present invention, it is possible to obtain a polyisobutylene polymer, an isobutylene-styrene block copolymer, and the like that are excellent in molecular weight distribution and dispersion degree.

In addition, in the above examples, α-methylstyrene was used as an ingredient for an initiator. However, it is needless to say that the present invention is applicable not only to an ingredient in which n is 1, but also to the ingredient in which n is two or more.

## Claims

1. A method for producing a chlorinated aromatic compound represented by general formula (2):
Ar(R¹CCH₃Cl)ₙ (2)
where Ar is an n-valent aromatic ring group, R¹ is a substituted or unsubstituted univalent aliphatic hydrocarbon group, and n is an integer from 1 to 5, comprising the steps of:
(a) performing chlorination reaction by stirring (i) an organic solution containing a compound represented by general formula (1) :
Ar(R¹C=CH₂)ₙ (1)
where Ar, R¹, and n are the same as those in general formula (2)
and (ii) an aqueous hydrochloric acid solution having a hydrochloric acid concentration of 30% by weight or higher;
(b) removing a water phase in part or whole from a reaction solution produced in the step (a) and then adding another aqueous hydrochloric acid solution having a hydrochloric acid concentration of 30% by weight or higher to the reaction solution,
the steps (a) and (b) being repeated until a chlorination reaction rate of the compound represented by the general formula (1) reaches a predetermined level.

2. The method as set forth in claim 1, wherein:
when a hydrochloric acid concentration of a water phase of the reaction solution becomes less than 30% by weight after the step (a), the water phase is removed in part or whole, and then another aqueous hydrochloric acid solution is added to the reaction solution so that a hydrochloric acid concentration of the water phase in the reaction solution becomes 30% by weight or higher.

3. The method as set forth in claim 1 or 2, wherein:
the addition of another aqueous hydrochloric acid solution is repeated until the chlorination reaction rate reaches 95mol% or more.

4. The method as set forth in any one of claims 1 to 3, wherein:
a hydrogen chloride gas is used additionally for chlorination.

5. A method for producing a chlorinated aromatic compound represented by general formula (2):
Ar(R¹CCH₃Cl)ₙ (2)
where Ar is an n-valent aromatic ring group, R¹ is a substituted or unsubstituted monovalent aliphatic hydrocarbon group, and n is an integer from 1 to 5,
comprising the successive steps of:
performing chlorination reaction by mixing (i) an organic solution containing a compound represented by general formula (1):
Ar(R¹C=CH₂)ₙ (1)
where Ar, R¹, and n are the same as those in general formula (2) and (ii) an aqueous hydrochloric acid solution having a hydrochloric acid concentration of 30% by weight or higher;
removing a water phase of the thus obtained reaction solution; and
bringing an oil phase of the reaction solution into contact with hydrogen chloride gas.

6. The method as set forth in any one of claims 1 to 5, wherein:
the compound represented by general formula (1) is α-methylstyrene, 1,4-diisopropenylbenzene, 1,3-diisopropenylbenzene, 1,2-diisopropenylbenzene, 1,3,5-triisopropenylbenzene, or 1,3-diisopropenyl-5-(tert-butyl) benzene.
